# EUROPÄISCHE PATENTSCHRIFT

(11) **EP 1 172 062 B1**
(45) Veröffentlichungstag und Bekanntmachung des Hinweises auf die Patenterteilung: **27.10.2004**
(21) Anmeldenummer: 00810615.5
(22) Anmeldetag: 13.07.2000
(51) Int. Cl.: A61B 3/024, A61B 3/06

(54) **Vorrichtung zum Prüfen visueller Funktionen des menschlichen Auges**
Device for testing visual functions of the human eye
Appareil pour tester les fonctions visuelles de l'oeil humain

(43) Veröffentlichungstag der Anmeldung: 16.01.2002
(73) Patentinhaber: Haag-Streit AG, 3098 Köniz (CH)
(72) Erfinder: Walther, Hansueli, 5400 Baden (CH); Gisler, Beat, 6340 Baar (CH)
(74) Vertreter: Groner, Manfred

(56) Entgegenhaltungen:
- EP-A- 0 650 693
- CH-A- 677 599
- US-A- 5 024 519
- US-A- 5 270 750
- US-A- 5 864 384

## Beschreibung

Die Erfindung betrifft eine Vorrichtung zum Prüfen visueller Funktionen des menschlichen Auges nach dem Oberbegriff des Anspruchs 1. Die Erfindung betrifft zudem Verwendungen dieser Vorrichtung.

Die US-A-5,024,519 offenbart eine Vorrichtung der genannten Art, bei welcher die Stimuli auf einem halbkreisförmigen Hohlkörper erzeugt werden, der direkt auf das Auge des Patienten aufgesetzt wird. Eine Innenseite des Hohlkörpers wird mit LEDs ausgeleuchtet. Bei dieser indirekten Projektion der Stimuli ist aufgrund der kurzen Distanz eine Fokussierung nicht möglich.

Eine weitere Vorrichtung ist im Stand der Technik aus der CH-A-677 599 bekannt geworden. Be dieser werden die Stimuli dem Patienten auf einer Fläche dargeboten. In diese Fläche kann eine Umfeldbeleuchtung eingeblendet werden, welche für den Patienten das ganze Umfeld der Beobachtungsachse gleichmässig ausleuchtet. Dazu ist in der optischen Achse ein gegen eine Zwischenebene gerichteter, teilweise lichtdurchlässiger Spiegel angeordnet, der einerseits das durch eine Linse durchtretende Licht durchlässt und andererseits das von der zweiten Lichtquelle erzeugte Licht gegen die Zwischenbildebene umlenkt. Die zweite Lichtquelle wird durch eine Halogenlampe gebildet. Da eine Halogenlampe sehr heiss wird, muss diese mittels eines Ventilators gekühlt werden.

Im Stand der Technik ist zudem ein Ganzfeld-Perimeter bkeannt geworden, mit dem eine flickernde Umfeldbeleuchtung erzeugbar ist. Das Flickerlicht wird mittels einer Halogenlampe und eines Schiebers erzeugt. Der Schieber wird bewegt und unterbricht das Licht der Halogenlampe mit einer bestimmten Frequenz. Bewegliche Schieber sind vergleichsweise aufwändig und defektanfällig.

Der Erfindung liegt die Aufgabe zugrunde, eine Vorrichtung der genannten Art zu schaffen, die auf einfache Weise die Erzeugung eins flickernden Umfeldes ermöglicht.

Die Aufgabe ist bei einer gattungsgemässen Vorrichtung gemäss Anspruch 1 gelöst. Der LED sind vorzugsweise zeitliche Elemente zugeordnet, die diese entweder konstant einschalten oder in wählbaren zeitlichen Verhältnissen flickern lassen. Die wählbaren Frequenzen sind bei einer LED im Wesentlich unbegrenzt, es können somit auch die Bereiche abgedeckt werden, in denen die sogenannte Verschmelzungsfrequenzen liegen.

Die erfindungsgemässe Vorrichtung hat den Vorteil, dass zur Erzeugung des flickernden Umfeldes auf bewegliche mechanische Teile verzichtet werden kann. Zudem erzeugen LEDs keine Verlustwärme, aktive Kühlung mittels Ventilator ist nicht erforderlich. Im Gegensatz zur Elektrophysiologie, wo teure Verstärker zur Auswertung der Flicker-ERGs benötigt werden, erfolgt die Antwort des Probanden mittels Antwortknopf.

Die zweite Lichtquelle weist wenigstens zwei auf unterschiedlichen Ebenen angeordnete LEDs und wenigstens ein Farbfilter auf. Dabei durchdringt das Licht der einen LED das Farbfilter und erzeugt ein entsprechend farbiges Umfeld. Hierbei besteht der wesentliche Vorteil, dass das Farbfilter fest installiert sein kann. Bisher mussten solche Farbfilter beweglich gelagert und bei Bedarf in den Strahlengang eingeschwenkt werden. Bei der erfindungsgemässen Vorrichtung wird für ein weisses Umfeld die LED eingeschaltet, deren Licht nicht durch das Farbfilter geht. Zur Erzeugung eines farbigen Umfeldes wird die andere LED eingeschaltet. Es sind hier auch Ausführungen mit meh-reren Farbfiltern möglich.

Weitere vorteilhafte Merkmale ergeben sich aus den abhängigen Patentansprüchen, der nachfolgenden Beschreibung und der Zeichnung.

Ein Ausführungsbeispiel der Erfindung wird nachfolgend anhand der Zeichnung näher erläutert. Es zeigen:
- Fig. 1: schematisch eine erfindungsgemässe Vorrichtung und
- Fig. 2: schematisch eine alternative Ausführung einer zweiten Lichtquelle.

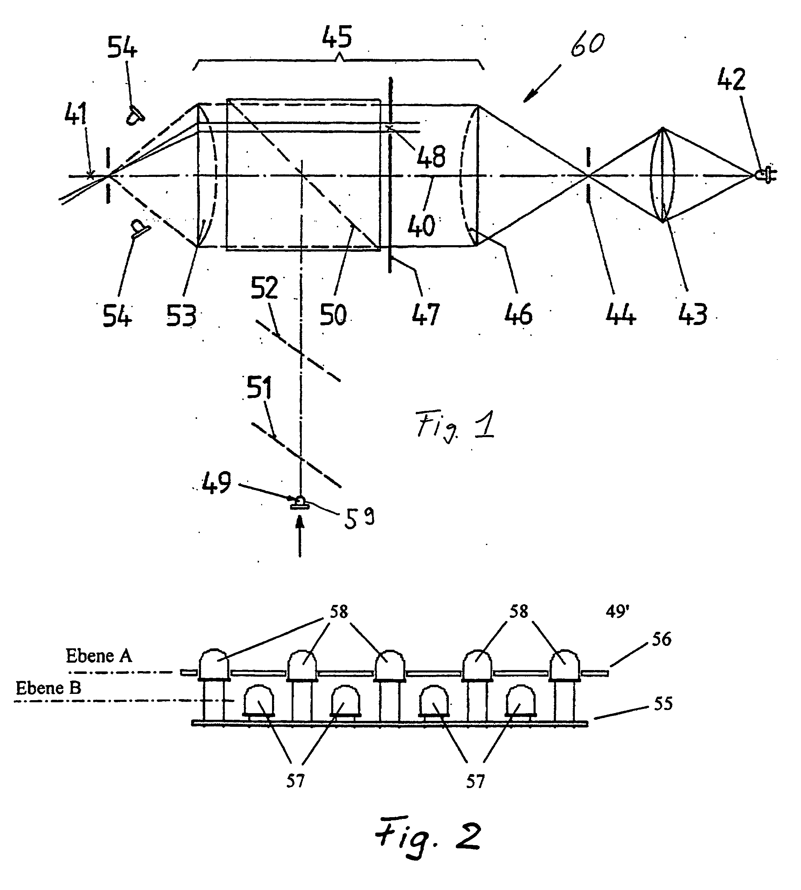

Das in Fig. 1 schematisch gezeigte Perimeter weist ein optisches System 60 auf, auf dessen optischer Achse 40 sich einerseits eine Beobachterstelle 41 und andererseits eine erste Lichtquelle 42 zur Erzeugung von Stimuli befinden. Stimuli sind kurze Lichtreize, welche dem Probanden an unterschiedlichen Stellen seines Gesichtsfeldes präsentiert werden. Das Licht der ersten Lichtquelle 42 wird durch eine Kondensorlinse 43 auf eine Blende 44 fokussiert und hinter der Blende 44 in einen parallelen Strahlengang 45 umgelenkt. Der Strahlengang 45 ist gegen die Blende 44 durch eine Feldlinse 46 und gegen die Beobachterstelle 41 durch eine Sammellinse 53 begrenzt. Die Beobachtungsstelle 41 ist nun so ausgebildet, dass die Sammellinse 53 das aus dem Strahlengang 45 austretende Licht auf das Zentrum der Pupille des Beobachterauges fokussiert. Sehfehler des Beobachterauges bleiben damit ohne Einfluss auf die Stimulusabbildung auf der Netzhaut. Im parallelen Strahlengang 45 ist rechtwinklig zur Beobachtungsachse 40 eine den ganzen Querschnitt sperrende Blende 47 mit einer den Stimulus hinsichtlich Form und Grösse begrenzenden Blendenöffnung 48 angeordnet. Die Blende 47 ist nach zwei zueinander rechtwinklig orientierten Richtungen in einer Ebene zur Beobachtungsachse 40 mittels eines hier nicht dargestellten und von einem ebenfalls nicht dargestellten Rechner gesteuerten Antrieb verschiebbar. Dadurch kann die Blendenöffnung 48 an jeden gewünschten und koordinatenmässig bestimmten Ort der Blendenebene verschoben werden. Die Darbietung der Stimuli erfolgt im parallelen Strahlengang 45 in der Blendenebene, von wo sie durch die Sammellinse 53 auf der Netzhaut des Beobachterauges abgebildet werden.

Die Umfeldbeleuchtung erfolgt mittels einer zweiten Lichtquelle 49, deren Licht über einen teilweise lichtdurchlässigen Umlenkspiegel 50 im Strahlengang 45 gegen die Sammellinse 53 gelenkt wird. Über zwei weitere teildurchlässige Umlenkspiegel 51 und 52 kann einerseits eine Fixationsmarke in das Umfeld eingeblendet und andererseits das Beobachterauge mittels einer Kamera überwacht werden.

Als zweite Lichtquelle 49 wird eine LED eingesetzt, die so angesteuert wird, dass einerseits ein kontinuierliches und andererseits ein flickerndes Umfeldlicht erzeugt werden kann. Ablaufstrategien lassen zu, dass neben der Intensität auch die Charakteristik des flickernden Umfeldlichts so verändert wird, dass die sogenannte Verschmelzungsfrequenz mittels Eingabelungsverfahren oder kontinuierlicher Annäherung ermittelt werden kann. Die Wahrnehmung des flickernden Umfelds wird vom Probanden mittels Antwortknopf signalisiert. Das Umfeldlicht kann weiss oder farbig sein. In Fig. 1 wird die zweite Lichtquelle 49 durch eine einzige LED gebildet. Es sind aber auch Ausführungen denkbar, bei welchen die zweite Lichtquelle 49 durch mehrere LEDs gebildet wird.

Bei der in Fig. 2 gezeigten Ausführung einer zweiten Lichtquelle 49' sind auf einem Träger 55 in unterschiedlichen Ebenen A und B mehrere LEDs 57 bzw. 58 angeordnet. Ein Farbfilter 56 ist auf dem Träger so befestigt, dass die LEDs 58 über diesem Filter und die LEDs 57 unterhalb dieses Filters 56 angeordnet sind. Um weisses Umfeldlicht zu erzeugen, werden lediglich die LEDs 58 betrieben. Um farbiges Umfeldlicht zu erhalten, werden die LEDs 57 aber nicht die LEDs 58 betrieben. Da die LEDs 57 wie ersichtlich unterhalb des Farbfilters 56 angeordnet sind, fällt ihr Licht durch dieses Farbfilter 56 und erhält dadurch die entsprechende Farbe. Für einen Farbwechsel müssen somit lediglich die LEDs 57 und 58 umgeschaltet werden. Das Farbfilter 56 kann fest eingebaut sein und muss somit für einen Farbwechsel nicht bewegt werden. Da die LEDs 57 - 59 im Betrieb im wesentlich keine Wärme abgeben, erübrigt sich eine aktive Kühlung der zweiten Lichtquelle 49 bzw. 49'.

## Patentansprüche

1. Vorrichtung zum Prüfen visueller Funktionen des menschlichen Auges, das sich an einer bestimmten Beobachtungsstelle (41) in Richtung einer Beobachtungsachse (40) orientiert, mit einer ersten Lichtquelle (42) zugeordneten Mitteln zur zeitlich gestaffelten Erzeugung von Stimuli an wählbaren Orten im Umfeld der Beobachtungsachse (40) und mit einer zweiten Lichtquelle (49, 49') zur Erzeugung von im Umfeld der Beobachtungsachse (40) gleichmässig verteiltem Licht, wobei die zweite Lichtquelle (49, 49') wenigstens eine LED (57-59) aufweist, **dadurch gekennzeichnet dass** die zweite Lichtquelle (49') wenigstens zwei LEDs (57, 58) und wenigstens ein Farbfilter (56) aufweist, wobei das Licht der einen LED (57) das Farbfilter (56) durchdringt und ein entsprechendes, farbiges Umfeldlicht erzeugt und dass die eine LED (57) hinter dem Farbfilter (56) und die andere LED (58) vor dem Farbfilter (56) angeordnet ist.

2. Vorrichtung nach Anspruch 1, **dadurch gekennzeichnet, dass** mehrere LEDs (57) mittels eines Farbfilters (56) ein farbiges Umfeldlicht erzeugen.

3. Verfahren zur Verwendung der Vorrichtung nach Anspruch 1, **dadurch gekennzeichnet, dass** die zweite Lichtquelle (49, 49') so ausgebildet ist, dass wahlweise ein stetiges oder ein flickerndes bzw. flimmerndes Umfeldlicht erzeugbar ist.

4. Verfahren nach Anspruch 3, **dadurch gekennzeichnet, dass** die Frequenz des flickernden Umfeldlichts wenigstens im Bereich üblicher Verschmelzungsfrequenzen veränderbar ist.

5. Verfahren nach Anspruch 3, **dadurch gekennzeichnet, dass** die Intensität des flickernden Umfeldlichts veränderbar ist.

## Claims

1. A device for testing visual functions of the human eye which is at a defined viewing point (41) and oriented along a viewing axis (40), comprising means associated with a first light source (42) for the temporally staggered generation of stimuli at selectable locations on the background of the viewing axis (40) and with a second light source (49, 49') for the generation of light that is distributed evenly on the background of the viewing axis (40) , wherein the second light source (49, 49') has at least one LED, **characterized in that** the second light source (49, 49') has at least two LEDs (57, 59) and at least one colored filter (56), wherein the light of one LED (57) goes through the coloured filter (56) and generates a corresponding coloured background light and that one LED (57) is placed behind the colored filter and the other LED (58) is placed in front of the coloured filter.

2. A device according to claim 1, **characterized in that** that several LEDs (57) go through a colored filter (56) and generate a corresponding colored background light.

3. Method for using the device according to claim 1, **characterized in that** the second light source (49, 49') is equipped so that a constant background light or a flickering background light can be generated alternatively.

4. Method for using the device according to claim 3, **characterized in that** the frequency of the flickering background light can be modified at least in the range of conventional critical flicker fusion frequencies.

5. Method according to claim 3, wherein the intensity of the flickering background light can be modified.

## Revendications

1. Dispositif de contrôle des fonctions visuelles de l'oeil humain qui s'oriente en un point d'observation (41) donné en direction d'un axe d'observation (40), comprenant des moyens associés à une première source de lumière (42) pour la production échelonnée dans le temps de stimuli en des endroits sélectionnables dans l'environnement de l'axe d'observation (40) et comprenant une deuxième source de lumière (49, 49') pour produire une lumière répartie régulièrement dans l'environnement de l'axe d'observation (40), la deuxième source de lumière (49, 49') présentant au moins une LED (57-59), **caractérisé en ce que** la deuxième source de lumière (49') présente au moins deux LED (57, 58) et au moins un filtre coloré (56), la lumière de l'une des LED (57) traversant le filtre coloré (56) et produisant une lumière ambiante colorée correspondante et que l'une des LED (57) est disposée derrière le filtre coloré (56) et l'autre LED (58) devant le filtre coloré (56).

2. Dispositif selon la revendication 1, **caractérisé en ce que** plusieurs LED (57) génèrent une lumière ambiante colorée au moyen d'un filtre coloré (56).

3. Procédé de mise en oeuvre du dispositif selon la revendication 1, **caractérisé en ce que** la deuxième source de lumière (49, 49') est configurée de manière à pouvoir générer, au choix, une lumière ambiante constante ou alors vacillante ou scintillante.

4. Procédé selon la revendication 3, **caractérisé en ce que** la fréquence de la lumière ambiante vacillante peut être modifiée au moins dans la plage des fréquences de fusion habituelles.

5. Procédé selon la revendication 3, **caractérisé en ce que** l'intensité de la lumière ambiante vacillante est modifiable.
